**Europäisches Patentamt**

**European Patent Office**

**Office européen des brevets**

(19)

(11) Veröffentlichungsnummer: **0 012 272**
**B1**

(12) # EUROPÄISCHE PATENTSCHRIFT

(45) Veröffentlichungstag der Patentschrift:
**30.12.81**

(51) Int. Cl.³: **A 61 K 33/14** // (A61K33/14, 31/70, 31/415, 31/405)

(21) Anmeldenummer: **79104734.3**

(22) Anmeldetag: **28.11.79**

(54) Protektive Lösung für Herz und Niere und Verfahren zu deren Herstellung.

(30) Priorität: **14.12.78 CH 12757/78**

(43) Veröffentlichungstag der Anmeldung:
**25.06.80 Patentblatt 80/13**

(45) Bekanntmachung des Hinweises auf die Patenterteilung:
**30.12.81 Patentblatt 81/52**

(84) Benannte Vertragsstaaten:
**AT BE DE FR GB NL SE**

(56) Entgegenhaltungen:
**Rote Liste, 1977/78, Editio Cantor,**
**Aulendorf/Württ., DE,**
**Ref. Nr. 51 031 B «Normofundin MD»**
**Ref. Nr. 51 116 B «Sterofundin HL»**
**Ref. Nr. 51 117 B «Tutofusin HG»**
**Ref. Nr. 51 110 B «Tutofusin D»**
**Ref. Nr. 51 103 B «Ringer Laktat pfrimmer»**

(73) Patentinhaber: **Dr. Franz Köhler Chemie KG, Neue Bergstrasse 5 - 7, D-6146 Alsbach/Bergstrasse (DE)**

(72) Erfinder: **Bretschneider, Hans-Jürgen Prof. Dr., Kolbergerstrasse 10, D-3406 Bovenden (DE)**

(74) Vertreter: **Zutter, Hans Johann Niklaus, EPROVA Aktiengesellschaft im Laternenacker 5, CH-8200 Schaffhausen (CH)**

## Protektive Lösung für Herz und Niere und Verfahren zu deren Herstellung

Die vorliegende Erfindung betrifft eine Lösung zur Protektion von Herz und Niere und anderen Organen gegenüber Schädigungen durch einen Durchblutungsstopp bei Operationen und Transplantation dieser Organe sowie das Verfahren zur Herstellung einer solchen Lösung.

Neue operative Verfahren zur Rekonstruktion schwerer angeborener Herzmissbildungen, Verbesserungen der Herz-Klappen-Prothesen und Fortschritte in der Chirurgie der Herzkranzgefässe haben Möglichkeiten zur vollständigen Wiederherstellung Schwerst-Kranker eröffnet. Während die Herz-Lungen-Maschinen-Technik die Ergebnisse komplizierter Herzoperationen kaum noch behindert, ist die Ischämie-Toleranz – die Toleranz des Organs gegenüber einer vollständigen Unterbrechung seiner Blut- und Sauerstoffversorgung bei Abschaltung aus dem Gesamtkreislauf – bis heute ein bedeutender limitierender Faktor.

Nach vollständiger Unterbrechung der Blut- und Sauerstoffzufuhr wird der Energiehaushalt und die Leistungsfähigkeit des Herzens rasch gestört. Daher wird von manchen Herzchirurgen die Methode der mehr oder weniger kontinuierlichen separaten Versorgung der Herzkranzgefässe mit Blut mittels spezieller Pumpen und Kanülen praktiziert. Diese Koronarperfusionstechnik ist jedoch auch mit schwerwiegenden Nachteilen verbunden: die Hauptäste der Herzkranzgefässe lassen sich nicht immer vollzählig kanülieren, die Koronar-Katheter und das mit Blut versorgte Organ behindern das Operationsfeld, und das schlagende Herz macht die Anwendung mikrochirurgischer Methoden mit dem Operationsmikroskop unmöglich.

Daher wurde frühzeitig die Technik der künstlichen Herzstillstandes mit totaler Unterbrechung der Blutzufuhr entwickelt. Das ischämisch stillgestellte Herz überlebt – je nach Vorschädigung und Temperatur – 20 bis 40 Minuten. Die dabei auftretende Erschöpfung der Energiereserven macht häufig eine längere Erholungsdauer mit Entlastung des Herzens durch die Herz-Lungen-Maschine und eine intensive Überwachung der in den ersten postoperativen Tagen stärker gefährdeten Patienten erforderlich. Ausserdem steht der Operateur bei diffizilen und komplizierten Eingriffen unter erheblichem Zeitdruck, da ein Überschreiten der sehr begrenzten Ischämie-Toleranzzeit unter allen Umständen vermieden werden muss.

Die limitierte Operationsdauer im rein ischämischen Herzstillstand war der Anlass für zahlreiche Versuche zur Verbesserung der Myocard-Protektion, z.B. durch Acetylcholin, durch Magnesiumsalze, durch Novocain und durch die von H.J. Bretschneider 1964 beschriebene Natriumarme, Calcium-freie, Novocain-haltige kardioplegische Lösung (H.J. Bretschneider: Überlebenszeit und Wiederbelebungszeit des Herzens bei Normo- und Hypothermie; Verh. Dtsch. Ges. Kreislaufforschg., 30 (1964), 11; H.J. Bretschneider, G. Hübner, D. Knoll, B. Lohr, H. Nordbeck und P.G. Spieckermann: Myocardial resistance and tolerance to ischemia: Physiological and biochemical basis; J. of Cardiovasc. Surg., 16 (1975), 241). Obwohl diese kardioplegische Lösung eine etwa doppelte Ischämie-Toleranzzeit gegenüber der beim rein ischämischen Herzstillstand garantiert, so ist es aufgrund der grossen Fortschritte der operativen Technik doch dringlich geworden, dass die vom Herzen gut tolerierte Ischämiedauer noch weiter verlängert wird und auf 100 bis 120 Minuten bei 30 °C ausgedehnt werden kann. Dabei muss die notwendige Erholungszeit möglichst kurz bleiben; nur etwa 10 Minuten, um die gesamte Dauer des Einsatzes der Herz-Lungen-Maschine so kurz als möglich zu halten.

Bis heute gibt es keine kardioplegische Lösung, welche diese hohe Ischämiedauer erlaubt.

Zweck der vorliegenden Erfindung ist, diese klaffende Lücke zu schliessen.

Zum besseren Verständnis der erfindungsgemäss beschriebenen neuen kardioplegischen Lösung sind nachstehend die Konsequenzen aus den Besonderheiten der Energie-Versorgung des Herzens dargestellt:

Der Energiebedarf des unter Ruhebedingungen arbeitenden Herzens beträgt – in Sauerstoff-Äquivalenten ausgedrückt – etwa 7 ml $O_2$ pro Minute und 100 g; bei maximaler Belastung kann der Energieumsatz vorübergehend bis zum 10-fachen Wert ansteigen. Der Energiebedarf des schlagenden Herzens kann auf folgende «Verbraucher» aufgeschlüsselt werden:

a) das kontraktile System im engeren Sinne,

b) die äussere Muskelmembran, welche die elektrophysiologischen Zündungsprozesse liefert,

c) die inneren Zellmembranen des sogenannten sarkoplasmatischen Retikulums, welche die Inaktivierung des kontraktilen Systems durch Reabsorption von Calcium-Ionen bewirken, und die Membran der Mitochondrien,

d) den verbleibenden Struktur-Erhaltungsstoffwechsel.

Das künstlich stillgelegte Herz hat dementsprechend einen um so geringeren Energiebedarf, je vollständiger das kontraktile System inaktiviert und erschlafft ist und je weitgehender die Ionen-Pumpen der äusseren Zellmembran und der inneren Membran-strukturen entlastet werden. Bei Eingriffen zur Drosselung des Energiebedarfes zum Zwecke der Verlängerung der Ischämie-Toleranz-Zeit des Herzens ist stets mit zu bedenken, dass sie rasch reversibel sein müssen und nicht zur Unterschreitung des Struktur-Erhaltungs-Stoffwechsels führen dürfen. Der sehr hohe Energie-Durchsatz im arbeitenden Herzen lässt sich nur auf aerobem Wege, d.h. durch «Verbrennung» der Substrate – im wesentlichen Glukose, Milchsäure und Fettsäuren – zu $CO_2$ und $H_2O$ mit Hilfe des Sauerstoffs sicherstellen. Ohne Sauerstoff – auf anaerobem Wege – können die Substrate nur partiell abgebaut werden und daher nur einen Bruchteil – etwa $1/13$ – der in ihnen enthaltenen Energie liefern. Der anaerobe Energie-Gewinn des Herzens basiert im wesentlichen auf dem Abbau der Speicher-Substanz Glykogen zu Milchsäure und weiteren dieser Glykolyse verwandten Prozessen. Daneben kann der Energie-Gehalt des Phospho-Kreatins und eines Teils des Adenosin-Triphosphats (ATP) genutzt werden. Ein ATP-Verlust von mehr als 25% der Norm hat jedoch eine erheblich verlängerte Erholungszeit zur Folge und ATP-Werte von etwa 60% der Norm bilden eine untere Grenze für die Wiederbelebbarkeit des Herzens unter klinischen Bedingungen.

Der anaerobe Energie-Gewinn im Herzen mittels der Glykogenolyse, d.h. der Spaltung des Glykogens zu Milchsäure, wird grundsätzlich durch drei Umstände limitiert:

1. Durch die begrenzten und nicht beliebig zu steigernden Glykogen-Vorräte.

2. Durch die zunehmende Selbsthemmung der Glykolyse aufgrund der Säuerung der Herzmuskelzellen durch die als Endprodukt anfallende Milchsäure.

3. Durch die im Laufe der Anaerobiose – trotz teilweiser Resynthese des ATP – offenbar zwangsläufig auftretende Abnahme des gesamten ATP-Gehaltes der Zelle, der für die weitere Unterhaltung der Glykolyse selbst einen Mindestwert nicht unterschreiten darf.

Die Ursachen der unzureichenden Effizienz der Glykolyse im Herzmuskel für die ATP-Regeneration sind im einzelnen unklar, sie hängen vielleicht z.T. mit dem unter 2. erwähnten Umstand zusammen und betreffen möglicherweise auch nur bestimmte Teile bzw. Kompartimente der Zelle. Analysen des Milchsäure- und Glykogen-Gehaltes des Herzens am Ende seiner Wiederbelebbarkeit haben gezeigt, dass die Höhe der Glykogen-Reserve im allgemeinen die Überlebenszeit nicht beschränkt; die Totenstarre tritt bereits ein, wenn noch erhebliche Glykogen-Vorräte vorhanden sind, sofern nicht die Ausgangswerte abnorm niedrig lagen oder die Milchsäure während der Anaerobiose ausgeschwemmt wurde. Daraus folgt der Gedanke, die Kapazität der Glykolyse (siehe 2.) durch Hinausschiebung der Säure-Selbst-Hemmung zu steigern und hierfür den relativ grossen Extrazellular-Raum des Herzens, der etwa 50% des intrazellulären Verteilungs-Raumes beträgt, zu nutzen. Als weitere Hypothese wurde der Gedanke verfolgt, über diesen Umweg – oder gegebenenfalls mit Hilfe zusätzlicher Massnahmen – auch die Effektivität der Glykolyse hinsichtlich des Wiederaufbaues von ATP, also den Wirkungsgrad der ATP-Resynthese (siehe 2.) zu verbessern. Daneben war das Prinzip einer Minimalisierung des Energiebedarfes aufrechterhalten bzw. noch konsequenter zu realisieren.

Es wurde gefunden, dass sich die Forderung der Nutzung des Extrazellularraumes des Herzens zur Abpufferung der sauren Endprodukte der Glykolyse, mit Hilfe des Puffersystems auf der Basis von Histidin + Histidin · Hydrochlorid unter Mitverwendung von Tryptophan verwirklichen lässt, wobei mit einer solchen protektiven Lösung eine Ischämie-Toleranzzeit erreicht wird, die einen Verbesserungsfaktor von etwa 4 gegenüber dem unbeeinflussten Herzen und einen Verbesserungsfaktor von etwa 2 gegenüber der 1964 publizierten Lösung, für alle funktionell entscheidenden Strukturen und Parameter des Herzens aufweist.

Entscheidend für die gleichzeitige Optimierung des Energiebedarfs der Zellmembranen und für ihre Milchsäure-Durchlässigkeit sind folgende Ionen-Parameter der neuen protektiven Lösung: der Gehalt an Natrium-, Kalium- und Magnesium-Ionen, das Produkt aus Kalium- und Chlorid-Ionen, der Säure-Grad der Lösung (pH-Wert) und ihre Osmolarität.

Ähnliche Anforderungen wie zur Verlängerung der Ischämie-Toleranz des Herzmuskels sind auch für die Nieren-Protektion erforderlich. Durch Anwendung der erfindungsgemässen Lösungen lässt sich daher auch eine Verbesserung der funktionellen Ischämie-Toleranz der Niere erzielen.

Demnach ist die neue, erfindungsgemässe protektive Lösung zur Verhinderung von Ischämie-Schäden an Herz und Nieren und weiteren Organen bei Operationen und Transplantationen dieser Organe unter Ausschluss des Blutkreislaufes, dadurch gekennzeichnet, dass sie aus einem Puffersystem auf der Basis von 150 ± 100 Millimol Histidin, 16 ± 10 Millimol Histidin · Hydrochlorid und 2 ± 1 Millimol Tryptophan pro Liter besteht, welches zusätzlich Alkali- und Erdalkaliionen sowie mindestens ein Polyol oder mindestens einen Zucker enthält, wobei die Summe der Kationen 25–80 Millival, die Osmolarität der Lösung 300–400 mosm beträgt und das pH der Lösung zwischen 6,6 und 7,6 liegt.

Gewöhnlich ist die Summe der Kationen auf 30–60 Millival und das pH der Lösung auf 7,0 bis 7,2 festgelegt.

Die erfindungsgemässen protektiven Lösungen sind ausserdem dadurch charakterisiert, dass sie ausser Histidin/Histidin · Hydrochlorid/Tryptophan-Puffer noch

| | | |
|---|---|---|
| Natriumchlorid | (15 ± | 5 Millimol), |
| Kaliumchlorid | (15 ± | 7 Millimol), |
| Lithiumchlorid | ( 3 ± | 3 Millimol), |
| Magnesiumchlorid | (15 ± | 7 Millimol), |
| Calciumchlorid | (0,1 ± 0,1 Millimol) | |
| Mannitol | (50 ± 50 Millimol) und/oder | |
| Fruktose | (50 ± 50 Millimol) und/oder | |
| Ribose | (50 ± 50 Millimol) und/oder | |
| Inosin | (50 ± 50 Millimol) | |

pro Liter Wasser enthalten.

Das Verfahren zur Herstellung einer Lösung zur Verhinderung von Ischämie-Schäden an Herz und Nieren und andern Organen bei Operationen und Transplantationen der Organe ist demnach dadurch gekennzeichnet, dass man durch Auflösen von 150 ± 100 Millimol Histidin, 16 ± 10 Millimol Histidin · Hydrochlorid und 2 ± 1 Millimol Tryptophan in 1 Liter Wasser ein Puffersystem aufbaut und diesem noch verhältnismässig geringe Mengen von Alkali- und Erdalkaliionen sowie mindestens ein Polyol oder mindestens einen Zucker zusetzt, wobei die Summe der zugesetzten Kationen auf 25–80 Millival und die Osmolarität der Lösung auf etwa 300–400 mosm gehalten und das pH der Lösung auf 6,6 bis 7,6 eingestellt wird. Die Summe der Kationen wird gewöhnliche auf 30–60 Millival und das pH der Lösung auf 7,0 bis 7,2 eingestellt.

Eine protektive wässrige Lösung wird bereitet, indem man die einzelnen Bestandteile in folgenden Mengen pro 1 Liter Wasser zusetzt: Histidin 150 ± 100 Millimol, Histidin·Hydrochlorid 16 ± 10 Millimol, Tryptophan 2 ± 1 Millimol, Natriumchlorid 15 ± 5 Millimol, Kaliumchlorid 15 ± 7 Millimol, Lithiumchlorid 3 ± 3 Millimol, Magnesiumchlorid 15 ± 7 Millimol, Calciumchlorid 0,1 ± 0,1 Millimol, Zucker oder Polyol 50 ± 50 Millimol, nämlich Mannitol 50 ± 50 Millimol und/oder Inosin 50 ± 50 Millimol und/oder Fruktose 50 ± 50 Millimol und/oder Ribose 50 ± 50 Millimol

Beispiel 1:
Protektive Lösung für Herz und Niere

Im Liter Aqua dest. rec. werden aufgelöst:

| | | | |
|---|---|---|---|
| Natriumchlorid: | 15 mmol | = | 30 mosm |
| Kaliumchlorid: | 10 mmol | = | 20 mosm |
| Lithiumchlorid: | 1 mmol | = | 2 mosm |
| Magnesiumchlorid: | 1 mmol | = | 3 mosm |
| Histidin: | 160 mmol | = | 160 mosm |
| Histidin·Hydrochlorid: | 16 mmol | = | 32 mosm |
| Tryptophan: | 2 mmol | = | 2 mosm |
| Mannitol: | 50 mmol | = | 50 mosm |
| | | | = 299 mosm |
| pH: | | = | 7,1 |

Beispiel 2:
Protektive Lösung für Herz, Niere sowie weitere Organe (Leber, Muskel, Zentralnervensystem)

Im Liter Aqua dest. rec. werden aufgelöst:

| | | |
|---|---|---|
| Natriumchlorid: | 15 mmol | = 30 mosm |
| Kaliumchlorid: | 20 mmol | = 40 mosm |
| Magnesiumchlorid: | 20 mmol | = 60 mosm |
| Calciumchlorid: | 0,1 mmol | = 0,3 mosm |
| Histidin: | 160 mmol | =160 mosm |
| Histidin·Hydrochlorid: | 16 mmol | = 32 mosm |
| Tryptophan: | 2 mmol | = 2 mmol |
| Fruktose: | | = 50 mosm |
| | | =374,3 mosm |
| pH | | 7,1 |

Die Lösungen werden mit Luft äquilibriert und sind damit weitgehend frei von Kohlensäure ($CO_2$).
Die bereits 1964 beschriebene Lösung 1 und die 1967 geringfügig modifizierte Lösung 2, welche den Stand der Technik charakterisieren, hatten folgende Zusammensetzungen:
Im Liter Aqua dest. rec. der kardioplegischen Lösungen 1 und 2 sind enthalten:

| | 1 | | 2 | |
|---|---|---|---|---|
| Natriumchlorid: | 5 | mmol | 12 | mmol |
| Kaliumchlorid: | 5 | mmol | 7 | mmol |
| Magnesiumchlorid: | – | | 1 | mmol |
| Glukose: | 5,5 | mmol | 11 | mmol |
| Procain: | 8,5 | mmol | 8,5 | mmol |
| Mannit: | 267,5 | mmol | 241 | mmol |
| Osmolarität: | 310 | mosm | 310 | mosm |
| pH: | 7,4 | | 7,4 | |

Der technische Fortschritt der protektiven Lösungen gemäss vorliegender Erfindung gegenüber dem Stande der Technik ist charaktierisiert
1. durch Minimalisierung des anaeroben Energie-Bedarfes des Organs, insbesondere während der ATP-Phase,
2. durch Optimierung der anaeroben Energie-Reserve und ihrer Nutzbarkeit,
3. durch Optimierung des Nutzeffektes der anaeroben glykolytischen Energiebereitstellung und
4. durch Optimierung der Lactatpermeation aus den Zellen in den gepufferten Extrazellularraum.
Mit den nachstehenden Tabellen 1 und 2 werden die wichtigsten Parameter vergleichend dargestellt. Sie enthalten die maximal nutzbaren Ischämiezeiten des Herzens (Tabelle 1) und die dabei auftretenden Lactat-Konzentrationen (Tabellen 2) im Temperaturbereich von 35 °C–5 °C. Sie demonstrieren den technischen Fortschritt gemäss Position 1–4 eindrücklich.

Tabelle 1
Maximale nutzbare Ischämiezeiten des Herzens bei verschiedenen Kardioplegiemethoden im Temperaturbereich von 35 °C–5 °C.

| Kardioplegische Lösung | Ischämiezeiten in Minuten bei | | | |
|---|---|---|---|---|
| | 35 °C | 25 °C | 15 °C | 5 °C |
| Nr. 1 (1964) | 70 | 110 | 256 | 415 |
| Nr. 2 (1967) | 62 | 142 | 282 | 435 |
| Beispiel 1 der vorl. Erfindung | 100 | 210 | 420 | 800 |

Tabelle 2
Lactat-Konzentrationen bei verschiedenen Kardioplegiemethoden im Temperaturbereich von 35 °C–5 °C in µ Mol/g Myokard-Feuchtgewicht.

| Kardioplegische Lösung | Lactat-Konzentrationen bei | | | |
|---|---|---|---|---|
| | 35 °C | 25 °C | 15 °C | 5 °C |
| Nr. 1 | 22 | 21 | 23 | 20 |
| Nr. 2 | 22 | 21 | 21 | 18 |
| erfindungsgemässe Lösung | 34 | 36 | 40 | 40 |

Es kann somit quantitativ gezeigt werden, dass sich mit der neuen protektiven Lösung unter gleichen Bedingungen etwa doppelt solange Ischämie-Toleranzzeiten erreichen lassen, wie mit dem bisher besten Verfahren, bzw. etwa vierfach solange Toleranzzeiten, verglichen mit dem reinischämischen Herzstillstand. Diese biochemischen Ergebnisse werden von funktionellen Wiederbelebungs-Versuchen ausgezeichnet bestätigt: diese zeigen, dass das gesamte Herz einschliesslich der differenten Strukturen des Erregungs-Bildungs- und Leitungssystems und des Herz-Nervensystems durch die erfindungsgemäss beschriebene protektive Lösung während eines totalen Stopps seiner Blut- und Sauerstoff-Versorgung bedeutend besser geschützt wird als durch alle bisher bekannten «herzprotektiven» Verfahren.

Die 4 entscheidenden Mechanismen zur Verlängerung der Ischämie-Toleranz des Herzens – Minimalisierung des anaeroben Energie-Bedarfes des Organs, Optimierung der anaeroben Energie-Reserve, Optimierung des Nutzeffektes der anaeroben Energiebereitstellung und Optimierung der Lactatpermeation aus den Zellen – sind wie nachgewiesen wird, auch an der Niere wirksam.

Eine Nierenprotektion, d.h. ein Schutz der Niere gegenüber einer Unterbrechung der Blut- und Sauerstoffzufuhr, ist sowohl für kompliziertere Operationen an der Niere in situ, als auch die Nierenkonservierung zwecks Transplantation von grosser Bedeutung.

Collins hat erstmals 1969 eine protektive Lösung für die Niere beschrieben und danach weiterentwickelt: Collins, G.M., Bravo-Shugarman, M.M. Terasaki, P.I.: Kidney preservation for transportation. Lancet II: 1219 (1969).

Collins, G.M., Halasz, N.A.: Current aspects of renal preservation. Urology (Suppl.) X:22–32 (1977). Collins, G.M., Halasz, N.A.: Clinical comparision of methods for cadaveric kidney preservation. J. Surg. Res. 24–396 (1978).

Im europäischen Verbundsystem für die Nierentransplantation mit Sitz in Holland wird eine etwas modifizierte Collins-Lösung verwendet, welche die Bezeichnung «Euro-Collins-Lösung» trägt.

Rezept für die Euro-Collins-Lösung:
Lösung A in 1 Liter Flasche enthaltend:

| | |
|---|---|
| $NaHCO_3$ | 0,84 g |
| $KH_2PO_4$ | 2,04 g |
| $K_2HPO_4$ | 7,40 g |
| KCl | 1,12 g |
| Glucose | 35,00 g |
| Aqua dest. ad | 980 ml |

Lösung B von 20 ml enthaltend:

| | |
|---|---|
| $NaHCO_3$ | 0,86 g |
| $KH_2PO_4$ | 2,08 g |
| $K_2HPO_4$ | 7,56 g |
| KCl | 1,14 g |
| Aqua dest. ad | 20 ml |

Lösung B wird vor dem Gebrauch mit Lösung A vereinigt. Die resultierende Euro-Collins-Lösung hat eine Gesamtosmolalität von 365 mosm und ein pH von 7,28 bei 10,5 °C.

Mit den bisher zur Verfügung stehenden Verfahren kann eine menschliche Niere ohne nachfolgende schwerwiegende Funktionsstörung nur für etwa 20–30 Minuten bei 30 °C von der Blutzufuhr abgesperrt werden. Die Nierenkonservierung mit der Euro-Collins-Lösung gewährt bei Temperaturen zwischen 0 und 5 °C nur über 20–24 Stunden einen hinreichenden Schutz.

Weit bessere Ergebnisse werden mit Pufferlösungen gemäss vorliegender Erfindung erzielt.

Durch eine selektive Perfusion mit einer erfindungsgemässen Pufferlösung über die Nierenarterie nach Unterbrechung der natürlichen Blutzufuhr während 8 Minuten bei 35 °C wird die Funktionstüchtigkeit, d.h. die nachträgliche Fähigkeit zur Harnausscheidung, weit besser erhalten als durch analoge Behandlung mit Euro-Collins-Lösung. Im Hundeversuch wurden mit der protektiven Lösung gemäss Beispiel 1 und mit Euro-Collins-Lösung die in der Tabelle 3 aufgeführten Ergebnisse erzielt.

Tabelle 3
Durchschnittliche Harnproduktion der Hundenieren in ml nach Wiedereröffnung der Blutzufuhr nach 60 Minuten langer Ischämiezeit bei ca. 35 °C.

| | ohne Diuretikum | | mit Mannit 20% | |
|---|---|---|---|---|
| | 0–10' | 10–30' | 30–60' | 60–90' |
| Beispiel 1 der vorliegenden Erfindung | 12 | 40 | 105 | 145 |
| Euro-Collins-Lösung | 4 | 4 | 33 | 35 |

In weiteren systematischen Versuchen an 42 Hunden wurde festgestellt, dass die Nieren bei entsprechender Protektion noch weit längere Ischämiezeiten ertragen.

In der Tabelle 4 sind die Resultate aus diesen vergleichenden Experimenten an Hunden, deren Nieren einerseits mit Euro-Collins-Lösung und anderseits mit der erfindungsgemässen Lösung gemäss Beispiel 2 behandelt wurden, aufgeführt. Die Behandlung bestand jeweils in einer 10 minütigen selektiven Perfusion mit der Pufferlösung über die Nierenarterie während einer Dauer von 10 Minuten bei 20 °C. Die Funktionstüchtigkeit wurde bei der Hälfte der Tiere nach zweistündiger Ischämiezeit und bei der zweiten Hälfte der Tiere nach dreistündiger Ischämiezeit durch Messung der Harnproduktionsfähigkeit gemessen. Wie aus der Tabelle 4 hervorgeht, sind nur die mit der erfindungsgemässen Lösung 2 behandelten Nieren in der Lage gewesen, nach Anschluss an die Blut- und damit Sauerstoffzufuhr sofort eine Harnproduktion aufzunehmen. Damit erreicht die Verbesserung der Nierenprotektion mittels der erfindungsgemässen Lösung 2 einen Faktor von 3–4 gegenüber dem bisher üblichen Verfahren.

Tabelle 4

Durchschnittliche Harnproduktion von Hunde-nieren unmittelbar nach Wiedereröffnung der Blutzufuhr.

Postischämische Diurese [ml/30 min p.i.] bei Protektion mit Euro-Collins-Lösung bzw. Lösung gemäss Beispiel 2 vorliegender Erfindung.

| Lösung | Euro-Collins-Lösung | | Lösung nach Beispiel 2 vorliegender Erfindung | |
|---|---|---|---|---|
| Ischämie-zeit | 0–30 min p.i. | 30–60 min p.i. | 0–30 min p.i. | 30–60 min p.i. |
| 2 Stunden | 0 ml | 0 ml | 20 ml (keine diuretische Vorbehandlung) | 30 ml |
| 3 Stunden | 0 ml | 0 ml | 60 ml (Vorbehandlung mit Furosemid) | 145 ml |

Diese stark verbesserte protektive Wirkung ermöglicht auch komplizierte und langwierige Operationen an der Niere in situ mit einer reichlichen Zeitreserve, wie z.B. die Entfernung grosser Ausgusssteine, die Resektion kleiner Tumoren, Cysten und Abzesse und die Operation komplizierter Nieren-Arterien-Stenosen.

Für Temperaturen zwischen 0–5 °C, wie sie für die Nierenkonservierung angewandt werden, ist mit einer entsprechenden Verlängerung der Ischä-mie-Toleranz gegenüber den bisher üblichen Verfahren zu rechnen. Damit wird die im Rahmen eines Nierentransplantations-Verfahrens zur Verfügung stehende Zeit, die bisher häufig unzureichend war, um ein entscheidendes Mass verlängert. Eine auf 2–3 Tage verlängerte Konservierungsdauer gibt die Möglichkeit, die Gewebsverträglichkeit sorgfältiger zu testen und die Trans-plantations-Operation besser vorzubereiten. Aus-serdem werden als Folge des verbesserten Funk-tionszustandes der Nieren postoperative Kompli-kationen reduziert.

Es ist zu erwarten, dass die an Herz und Niere bewiesenen protektiven Wirkungen auch an ande-ren Organen – Leber, Muskel, Zentralnervensy-stem – nutzbar sind.

Im europäischen Recherchenbericht wurden einige Infusionslösungen aus der ROTEN LISTE zitiert, insbesondere
Ringer-Laktat-Pfrimmer [R]
Tutofusin [R] D & HG5
Sterofundin[R] HL-5/HL-10 und
Normofundin[R] MD.

Diese Elektrolyt- und Aminosäurelösungen werden ausschliesslich zur Infusion verwendet, d.h. sie werden bei erhaltener Herztätigkeit in den Kreislauf eingeführt. Sie sollen die Herztätigkeit stimulieren und verbessern und/oder das endogene Stickstoffminimum aufrecht erhalten. Ihre Zusammensetzung ist demgemäss völlig ungeeignet zur Protektion von stillstehenden, vom natürlichen Blutkreislauf getrennten Organen, insbesondere von Herz und Niere gegenüber Schädigungen durch den Durchblutungsstopp, der bei Operationen und Transplantationen erforderlich ist.

Wenn auch gewisse Bestandteile sowohl bei den vorbekannten Infusionslösungen als auch den erfindungsgemässen protektiven Lösungen für stillstehende Organe wie Herz und Niere verwendet werden, so ist doch die Zusammensetzung der vorbekannten Infusionslösungen grundsätzlich verschieden von denen der erfindungsgemässen protektiven Lösungen für stillstehende Organe.

Die protektiven Lösungen für stillstehende Organe bestehen im Prinzip aus einem Puffersystem auf der Basis von Histidin/Histidin · HCl. Sie ent-halten noch etwas Tryptophan und zusätzlich Al-kali- und Erdalkali-Ionen und gewöhnlich etwas Zucker oder einen Zuckeralkohol. Die entgegengehaltene Ringer-Laktat-Pfrimmer[R]-Infusionslösung enthält dagegen nur Alkali- und Erdalkali-Ionen und darüber hinaus Milchsäure als Natriumlaktat. Ähnliches gilt für die entgegengehaltenen Infusionslösungen Tutofusin[R] D und HG5 sowie Sterofundin[R] HL-5 und HL-10, welche kein Laktat dafür jedoch Sorbit, Glukose oder Fructose enthalten.

Einzig die Normofundin[R]-Lösung MD enthält zusätzlich sämtliche für den Menschen unentbehrlichen (essentiellen) Aminosäuren und daher zwangsläufig auch etwas Histidin und Tryptophan. Die essentiellen Aminosäuren im Normo-fundin[R] sind Isoleucin, Leucin, Lysin, Methionin, Phenylalanin, Threonin, Tryptophan, Valin, Arginin, Histidin, Glycin, Alanin, Prolin, Asparaginsäure, Cystin, Glutaminsäure, Serin, Tyrosin; dazu kommt noch Asparagin Ornithin. Infusionen von Normofundin[R] werden zur Aufrechterhaltung des endogenen Stickstoffminimums nach Operationen oder Traumen verwendet.

Keine der entgegengehaltenen Lösungen enthalten das für die protektiven Lösungen für stillstehende Organe charakteristische Puffersystem Histidin:Histidin · Hydrochlorid. Zum Vergleich haben wir die Zusammensetzung von Normofun-din[R] und einer typischen erfindungsgemässen protektiven Lösung zur Verhinderung von Ischä-mieschäden einander gegenübergestellt.

Anschliessend werden auch die Zusammensetzungen der übrigen zitierten Infusionslösungen aufgeführt.

Vergleich:

| Normofundin(R) MD 1 Liter enthält | | Protektive Lösung für Herz und Niere gemäss Beispiel 1 1 Liter enthält: | |
|---|---|---|---|
| | Millimol | | Millimol |
| Isoleucin | 5,79 | Histidin | 160 |
| Leucin | 10,21 | Histidin · Hydrochlorid | 16 |
| Lysin · HCl | 5,75 | Tryptophan | 2 |
| Methionin | 3,8 | Mannitol | 50 |
| Phenylalanin | 4,66 | Natriumchlorid | 15 |
| Threonin | 5,12 | Kaliumchlorid | 10 |
| Tryptophan | 1,32 | Lithiumchlorid | 1 |
| Valin | 6,14 | Magnesiumchlorid | 1 |
| Arginin | 7,92 | | |
| Histidin · HCl | 5,0 | Die erfindungsgemässe Lö- | |
| Glycin | 15,85 | sung enthält 33 mal mehr Hi- | |
| Alanin | 23,0 | stidin/Histindin · HCl, 12 mal | |
| Prolin | 11,55 | weniger Zucker und Zuckeral- | |
| Asparaginsäure | 1,5 | kohole (Polyole), | |
| Asparagin | 3,70 | 5 mal weniger Na+ und 12 mal | |
| Cystin | 0,33 | weniger K+ als Normofundin | |
| Glutaminsäure | 4,69 | (R) und nur 2 statt 20 verschie- | |
| Ornithin · HCl | 2,85 | dene Aminosäuren. | |
| Serin | 3,43 | Die Zusammensetzung der | |
| Tyrosin | 1,10 | beiden Lösungen ist also | |
| Sorbit | 274,5 | grundsätzlich verschieden. | |
| Xylit | 328,6 | | |
| Natriumacetat | 32 | | |
| Kaliumchlorid (9,02 g) | 121 | | |
| Zinkacetat | 0,07 | | |
| Natriumchlorid | 47,91 | | |
| Calciumchlorid | 2,65 | | |
| Magnesiumchlorid | 3,00 | | |

| Sterofundin(R) HL 1 Liter enthält: | | Turofusin(R) D 1 Liter enthält: | |
|---|---|---|---|
| | Millimol | | Millimol |
| Natriumchlorid | 50,13 | Natriumchlorid | 95 |
| Kaliumchlorid | 2 | Natriumacetat | 45 |
| Magnesiumchlorid | 0,5 | Calciumchlorid | 2,5 |
| Calciumlactat | 1,25 | Magnesiumchlorid | 1,5 |
| Natriumlactat | 20 | Sorbit | 86 |
| Fructose | 278 | | |

| Tutofusin(R) HG 1 Liter enthält: | | Ringer-Laktat-Pfrimmer(R) 1 Liter enthält: | |
|---|---|---|---|
| | Millimol | | Millimol |
| Natriumchlorid | 70 | Natriumchlorid | 102 |
| Kaliumchlorid | 2,5 | Kaliumchlorid | 4 |
| Calciumchlorid | 1,65 | Calciumchlorid | 1,8 |
| Magnesiumchlorid | 0,75 | Magnesiumchlorid | 1 |
| Glukose-Monohydrat | 278 | Natriumlaktat | 55 |

Gegenüber den erfindungsgemässen protektiven Lösungen für nicht am Blutkreislauf angeschlossene Organe fehlt bei allen zitierten Infusionslösungen das Puffersystem Histidin/Histidin HCl (+ Tryptophan).

Verglichen mit Beispiel 1 enthalten die zitierten Aminosäure-freien Infusionslösungen 5–10 mal mehr Na+ und bedeutend weniger K+.

Osmolarität, Osmolalität (aus Wissenschaftliche Tabellen, Documenta Geigy, Thieme 1975, S. 265) «Unter Osmolarität (in Osmol (osm) pro Liter Lösung) bzw. Osmolalität (in Osmol (osm) pro Kilogramm Wasser) versteht man diejenige Molarität bzw. Molalität, welche eine ideale Lösung eines nicht dissoziiernden Stoffes besitzen müsste, um denselben osmotischen Druck auszuüben wie die betrachtete Lösung. Der Begriff ist der physikalisch-chemischen Literatur fremd, wird aber in der biologisch-medizinischen Literatur vielfach benützt.»

mosm = milliosm = 1/1000 osm

**Patentansprüche**

1. Protektive Lösung zur Verhinderung von Ischämie-Schäden an Herz und Nieren und weiteren Organen bei Operationen und Transplantationen dieser Organe unter Ausschluss des Blutkreislaufes, dadurch gekennzeichnet, dass sie aus einem Puffersystem auf der Basis von 150 ± 100 Millimol Histidin, 16 ± 10 Millimol Histidin·Hydrochlorid und 2 ± 1 Millimol Tryptophan pro Liter besteht, welches zusätzlich Alkali- und Erdalkali-ionen sowie mindestens ein Polyol oder mindestens einen Zucker enthält, wobei die Summe der Kationen 25–80 Millival, die Osmolarität der Lösung 300–400 mosm beträgt und das pH der Lösung zwischen 6,6 und 7,6 liegt.

2. Protektive Lösung gemäss Patentanspruch 1, dadurch gekennzeichnet, dass die Summe der Kationen 30–60 Millival beträgt und das pH der Lösung bei 7,0 bis 7,2 liegt.

3. Protektive Lösung gemäss Patentansprüchen 1 und 2, dadurch gekennzeichnet, dass sie ausser Histidin/Histidin·Hydrochlorid/Tryptophan-Puffer noch

| | |
|---|---|
| Natriumchlorid | (15 ± 5 Millimol), |
| Kaliumchlorid | (15 ± 7 Millimol), |
| Lithiumchlorid | ( 3 ± 3 Millimol), |
| Magnesiumchlorid | (15 ± 7 Millimol), |
| Calciumchlorid | (0,1 ± 0,1 Millimol) |
| Mannitol | (50 ± 50 Millimol) und/oder |
| Fruktose | (50 ± 50 Millimol) und/oder |
| Ribose | (50 ± 50 Millimol) und/oder |
| Inonsin | (50 ± 50 Millimol) |
| pro Liter Wasser enthält | |

4. Verfahren zur Herstellung einer Lösung zur Verhinderung von Ischämie- Schäden an Herz und Nieren und andern Organen bei Operationen und Transplantationen der Organe, dadurch gekennzeichnet, dass man durch Auflösen von 150 ± 100 Millimol Histidin, 16 ± 10 Millimol Histidin·Hydrochlorid und 2 ± 1 Millimol Tryptophan pro Liter Wasser ein Puffersystem aufbaut und diesem noch verhältnismässig geringe Mengen von Alkali- und Erdalkaliionen sowie mindestens ein Polyol oder mindestens einen Zucker zusetzt, wobei die Summe der zugesetzten Kationen auf 25–80 Millival und die Osmolarität der Lösung auf 300–400 mosm gehalten und das pH der Lösung auf 6,6 bis 7,6 eingestellt wird.

5. Das Verfahren nach Patentanspruch 4, dadurch gekennzeichnet, dass die Summe der Kationen auf 30–60 Millival und das pH der Lösung auf 7,0 bis 7,2 eingestellt wird.

6. Verfahren nach Anspruch 4, dadurch gekennzeichnet, dass man die einzelnen Bestandteile in folgenden Mengen pro 1 Liter Wasser zusetzt: Histidin 150 ± 100 Millimol, Histidin·Hydrochlorid 16 ± 10 Millimol, Tryptophan 2 ± 1 Millimol, Natriumchlorid 15 ± 5 Millimol, Kaliumchlorid 15 ± 7 Millimol, Lithiumchlorid 3 ± 3 Millimol, Magnesiumchlorid 15 ± 7 Millimol, Calciumchlorid 0,1 ± 0,1 Millimol, Mannitol 50 ± 50 Millimol und/oder Fruktose 50 ± 50 Millimol, Ribose 50 ± 50 Millimol und/oder Inosin 50 ± 50 Millimol.

**Revendications**

1. Solution protectrice pour la prévention des lésions causées par l'ischémie sur le cœur, le rein et d'autres organes dans les opérations et transplantations de ces organes lorsqu'ils sont coupés de la circulation sanguine, caractérisée en ce qu'elle se compose d'un système tampon à base de 150 ± 100 mMoles d'histidine, 16 ± 10 mMoles de chlorhydrate d'histidine et 2 ± 1 mMoles de tryptophane par litre, qui contient en outre des ions alcalins et alcalino-terreux ainsi qu'au moins un polyol ou au moins un sucre, la somme des cations s'élevant à 25–80 Millival, l'osmolarité de la solution à 300–400 mosm et le pH de la solution se situant entre 6,6 et 7,6.

2. Solution protectrice selon la revendication 1, caractérisée en ce que la somme des cations s'élève à 30–60 Millival et le pH de la solution à 7,0 à 7,2.

3. Solution protectrice selon les revendications 1 et 2, caractérisée en ce qu'elle contient, outre le tampon histidine/chlorhydrate d'histidine/tryptophane,

| | |
|---|---|
| Chlorure de sodium | (15 ± 5 Millimol), |
| Chlorure de potassium | (15 ± 7 Millimol), |
| Chlorure de lithium | ( 3 ± 3 Millimol), |
| Chlorure de magnésium | (15 ± 7 Millimol), |
| Chlorure de calcium | ( 0,1 ± 0,1 Millimol), |
| Mannitol | (50 ± 50 Millimol) et/ou |
| Fructose | (50 ± 50 Millimol) et/ou |
| Ribose | (50 ± 50 Millimol) et/ou |
| Inosine | (50 ± 50 Millimol) |
| par litre d'eau | |

4. Procédé de préparation d'une solution destinée à prévenir les lésions provoquées par l'ischémie sur le cœur, les reins et d'autres organes dans les opérations et les transplantations des organes, caractérisé en ce qu'on construit un système tampon en dissolvant 150 ± 100 mMoles d'histidine, 16 ± 10 mMoles de chlorhydrate d'histidine et 2 ± 1 mMoles de tryptophane par litre d'eau et en ce qu'on ajoute encore à celui-ci des quantités relativement faibles d'ions alcalins et alcalino-terreux ainsi qu'au moins un polyol ou au moins un sucre, la somme des cations ajoutés étant maintenue à 25–80 Millival et l'osmolarité de la solution à 300–400 mosm et le pH de la solution étant réglé à 6,6 à 7,6.

5. Procédé selon la revendication 4, caractérisé en ce que la somme des cations est fixée à 30–60 Millival et le pH de la solution à 7,0 à 7,2.

6. Procédé selon la revendication 4, caractérisé en ce qu'on utilise les différents composants dans les quantités suivantes pour 1 litre d'eau; histidine 150 ± 100 mMoles, chlorhydrate d'histidine 16 ± 10 mMoles, tryptophane 2 ± 1 mMoles, chlorure de sodium 15 ± 5 mMoles, chlorure de potassium 15 ± 7 mMoles, chlorure de lithium 3 ± 3 mMoles, chlorure de magnésium 15 ± 7 mMoles, chlorure de calcium 0,1 ± 0,1 mMole, mannitol 50 ± 50 mMoles et/ou fructose 50 ± 50 mMoles et/ou inosine 50 ± 50 mMoles.

**Claims**

1. Protective solution for preventing ischaemia damage to the heart and kidneys and other organs in the course of operations and transplantations of these organs whilst the blood circulation is shut out, characterised in that it consists of a buffer system based on 150 ± 100 millimols of histidine, 16 ± 10 millimols of histidine hydrochloride and 2 ± 1 millimols of tryptophane per litre, which additionally contains alkali metal ions and alkaline earth metal ions as well as a polyol or a sugar, the sum of the cations being 25–80 milliequivalents, the osmolarity of the solution being 300–400 mosm and the pH of the solution being between 6.6 and 7.6.

2. Protective solution according to Patent Claim 1, characterised in that the sum of the cations is 30–60 milliequivalents and the pH of the solution is 7.0 to 7.2.

3. Protective solution according to Patent Claims 1 and 2, characterised in that in addition to histidine/histidine hydrochloride/tryptophane buffer it also contains the following, per litre of water:

| | |
|---|---|
| sodium chloride | (10 ± 5 millimols) |
| potassium chloride | (15 ± 7 millimols) |
| lithium chloride | ( 3 ± 3 millimols) |
| magnesium chloride | (15 ± 7 millimols) |
| calcium chloride | ( 0.1 ± 0.1 millimol) as well |

as at least 50 millimols of a polyol or sugar, namely

| | |
|---|---|
| mannitol | (50 ± 50 millimols) and/or |
| fructose | (50 ± 50 millimols) and/or |
| ribose | (50 ± 50 millimols) and/or |
| inosine | (50 ± 50 millimols). |

4. Process for the preparation of a solution for preventing ischaemia damage to the heart and kidneys and other organs during operations and transplantations of the organs, characterised in that a buffer system is made up by dissolving 150 ± 100 millimols of histidine, 16 ± 10 millimols of histidine hydrochloride and 2 ± 1 millimols of tryptophane per litre of water and, additionally, relatively small amounts of alkali metal ions and alkaline earth metal ions as well as a polyol or a sugar are added to this system, the sum of the added cations being kept at 25–80 milliequivalents and the osmolarity of the solution at 300–400 mosm, and the pH of the solution being adjusted to 6.6–7.6.

5. The process according to Patent Claim 4, characterised in that the sum of the cations is adjusted to 30–60 milliequivalents and the pH of the solution to 7.0–7.2.

6. Process according to Claim 4, characterised in that the individual constituents are added in the following amounts per litre of water: histidine 150 ± 100 millimols, histidine hydrochloride 16 ± 10 millimols, tryptophane 2 ± 1 millimols, sodium chloride 15 ± 5 millimols, potassium chloride 15 ± 7 millimols, lithium chloride 3 ± 3 millimols, magnesium chloride 15 ± 7 millimols, calcium chloride 0.1 ± 0.1 millimol, sugar or polyol 50 millimols, namely mannitol 50 ± 50 millimols and/or fructose 50 ± 50 millimols, ribose 50 ± 50 millimols and/or inosine 50 ± 50 millimols.